# EUROPEAN PATENT SPECIFICATION

(11) **EP 3 288 976 B1**
(45) Date of publication and mention of the grant of the patent: **08.04.2020**
(21) Application number: 16720685.3
(22) Date of filing: 27.04.2016
(51) Int. Cl.: C07K 16/22

(54) **TREATMENT OF FIBRODYSPLASIA OSSIFICANS PROGRESSIVA**
BEHANDLUNG VON FIBRODYSPLASIA OSSIFICANS PROGRESSIVA
TRAITEMENT DE LA FIBRODYSPLASIE OSSIFIANTE PROGRESSIVE

(30) Priority: 29.04.2015 US 201562154617 P; 30.04.2015 US 201562155427 P
(43) Date of publication of application: 07.03.2018
(73) Proprietor: Regeneron Pharmaceuticals, Inc., Tarrytown, NY 10591 (US)
(72) Inventor: HATSELL, Sarah J., Tarrytown, New York 10591 (US); ECONOMIDES, Aris N., Tarrytown, New York 10591 (US); IDONE, Vincent J., Tarrytown, New York 10591 (US)
(74) Representative: Mewburn Ellis LLP
(86) International application number: PCT/US2016/029585
(87) International publication number: WO 2016/176341

(56) References cited:
- WO-A1-2013/063536
- WO-A1-2015/152183
- FREDERICK S KAPLAN ET AL: "From mysteries to medicines: drug development for fibrodysplasia ossificans progressiva", EXPERT OPINION ON ORPHAN DRUGS, vol. 1, no. 8, 1 August 2013 (2013-08-01), pages 637-649, XP055228116, DOI: 10.1517/21678707.2013.825208
- SONGTING SHI ET AL: "Antisense-Oligonucleotide Mediated Exon Skipping in Activin-Receptor-Like Kinase 2: Inhibiting the Receptor That Is Overactive in Fibrodysplasia Ossificans Progressiva", PLOS ONE, vol. 8, no. 7, 4 July 2013 (2013-07-04), page e69096, XP055228071, DOI: 10.1371/journal.pone.0069096
- PAUL B YU ET AL: "BMP type I receptor inhibition reduces heterotopic ossification", NATURE MEDICINE, NATURE PUBLISHING GROUP, NEW YORK, NY, US, vol. 14, no. 12, 1 December 2008 (2008-12-01), pages 1363-1369, XP008152158, ISSN: 1078-8956, DOI: 10.1038/NM.1888 [retrieved on 2008-11-30]
- S. J. HATSELL ET AL: "ACVR1R206H receptor mutation causes fibrodysplasia ossificans progressiva by imparting responsiveness to activin A", SCIENCE TRANSLATIONAL MEDICINE, vol. 7, no. 303, 2 September 2015 (2015-09-02), pages 303ra137-303ra137, XP055229101, Washington, DC ISSN: 1946-6234, DOI: 10.1126/scitranslmed.aac4358

## Description

### BACKGROUND

Fibrodysplasia Ossificans Progressiva (FOP) is an autosomal dominant disorder characterized by early onset, episodic and progressive ossification of skeletal muscle and associated connective tissue. FOP is driven by mutations in the intracellular domain of ACVR1 (ALK2), with the great majority altering Arginine 206 to Histidine (R206H) (Pignolo, R.J. et al. 2011, Orphanet J. Rare Dis.6:80)*.* ACVR1 is a type I receptor for bone morphogenic proteins (BMPs). The R206H mutation, among others, is believed to increase the sensitivity of the receptor to activation and render it more resistant to silencing. No effective medical therapy is known for FOP. Targeting of ACVR1/ALK2 has been suggested as a treatment of FOP (Kaplan et al. 2013, Expert Opinion on Orphan Drugs 1:637).

### SUMMARY OF THE CLAIMED INVENTION

The scope of the present invention is defined by the claims. Any information that does not fall within the claims is provided for information only. The disclosure concerns methods of treating Fibrodysplasia Ossificans Progressiva (FOP), comprising administering to a subject having FOP an effective regime of an antibody against Activin B, BMP9 or BMP10. In some methods, the antibody is chimeric, veneered, humanized or human antibody. In some methods, the antibody is an intact antibody. In some methods, the antibody is a human kappa IgG1 antibody. In some methods, a combination of antibodies against two or more of Activin B, BMP9 and BMP10 is administered. In some methods, the antibody is administered in combination therapy with an ACVR1, ACVR2A, or ACVR2B extracellular domain-Fc fusion protein or an antibody against Activin A.

The invention further relates to the use of an antibody against Activin B in the manufacture of a medicament for treating Fibrodysplasia Ossificans Progressiva (FOP). Optionally, the antibody is chimeric, veneered, humanized or human antibody. Optionally, the antibody is an intact antibody. Optionally, the antibody is a human kappa IgG1 antibody. Optionally, a combination of antibodies against two or more of Activin B plus, BMP9 or BMP10 is administered. Optionally, the antibody is administered in combination therapy with an ACVR1, ACVR2A, or ACVR2B extracellular domain-Fc fusion protein or an antibody against Activin A.

### BRIEF DESCRIPTION OF THE FIGURES

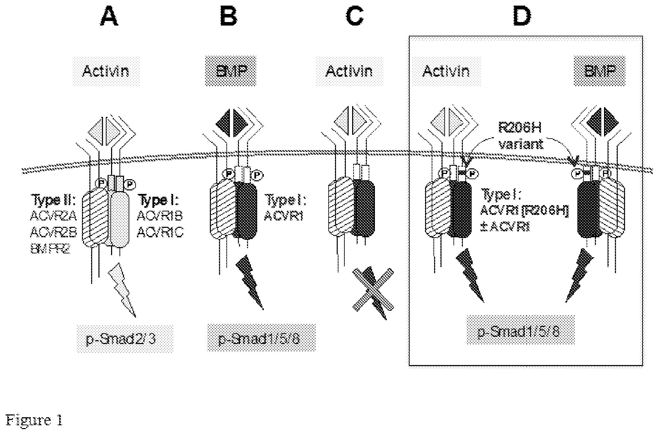

Figs. 1A-D show a schematic showing activation of ACVR1 R206H by the non-cognate ligand Activin B and a cognate BMP ligand. Fig. 1A shows Activin B signaling via the type I receptors ACVR1B/1C and Smad2/3 phosphorylation, and sharing type II receptors (ACVR2A, ACVR2B, and BMPR2) with BMPs. Fig. 1B shows ACVR1 together with the type II receptors, recognizing BMPs and stimulates phosphorylation of Smad1/5/8. In Fig. 1C, ACVR1, together with the type II receptors, binds Activin B but the resulting complex does not stimulate phosphorylation of Smadl/5/8; instead, Activin B acts as a competitive inhibitor of canonical BMP-mediated signaling through ACVR1. In Fig. 1D the R206H variant of ACVR1 responds to Activin B, inducing Smadl/5/8 phosphorylation, just like a BMP, effectively converting the ACVR2•ACVR1•Activin complex from a 'dead end' complex into a signaling complex.

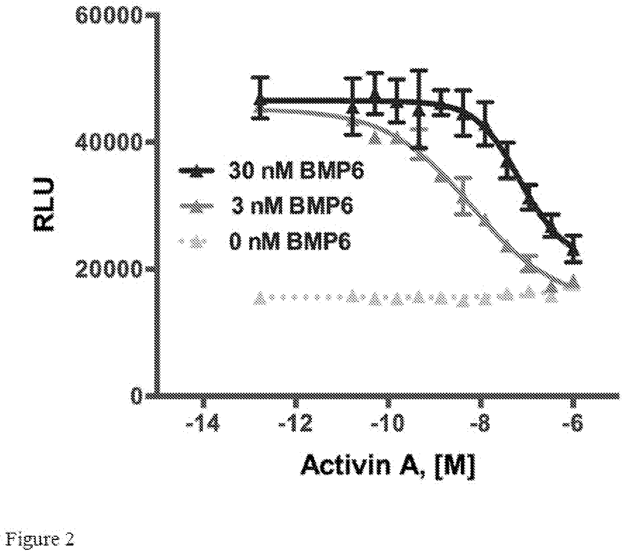

Fig. 2 shows Activin A inhibits BMP6 signaling via ACVR1.

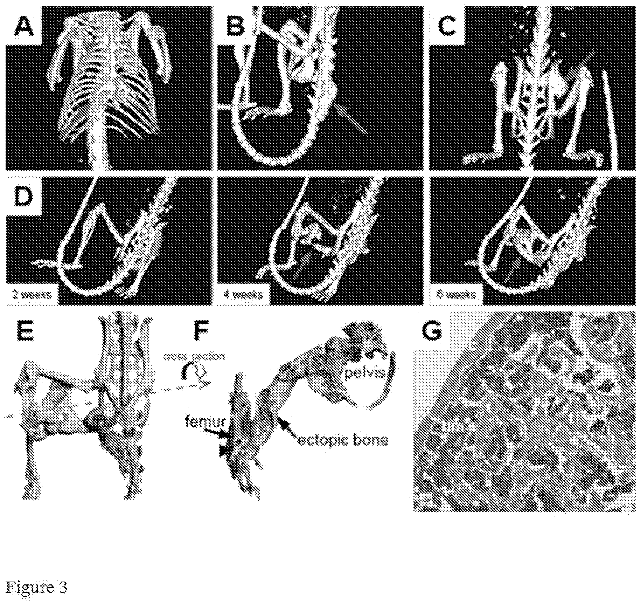

Figs. 3A-C show heterotopic bone formation in *Acvr1*^{*[R206H]FlEx*/+} ; *Gt(ROSA26)Sor*^{*CreERT2*/+} mice without treatment in the (A) sternum, (B) caudal vertebrae and (C) hip joint. Fig. 3D shows ectopic bone growth formed between 2 and 4 weeks after tamoxifen injection and can occur distal to the existing skeleton. Fig. 3E shows an ex-vivo µCT image of an ectopic bone lesion from the dorsal view showing bridging from the femur to the pelvis. Fig. 3F shows a transverse view through the ectopic bone shows that the newly formed bone has both cortical and trabecular like structures. Fig. 3G shows H&E stained histological sections of ectopic bone lesion demonstrates cortical (c) and trabecular bone (t) like structures and bone marrow (bm).

### DEFINITIONS

Therapeutic agents such as antibodies or ECD-Fc fusion proteins are typically provided in isolated form. This means that an agent is typically at least 50% w/w pure of interfering proteins and other contaminants arising from its production or purification, but does not exclude the possibility that the agent is combined with an excess of pharmaceutical acceptable carrier(s) or other vehicle intended to facilitate its use. Sometimes agents are at least 60, 70, 80, 90, 95 or 99% w/w pure of interfering proteins and contaminants from production or purification.

For purposes of classifying amino acids substitutions as conservative or nonconservative, amino acids are grouped as follows: Group I (hydrophobic side chains): met, ala, val, leu, ile; Group II (neutral hydrophilic side chains): cys, ser, thr; Group III (acidic side chains): asp, glu; Group IV (basic side chains): asn, gln, his, lys, arg; Group V (residues influencing chain orientation): gly, pro; and Group VI (aromatic side chains): trp, tyr, phe. Conservative substitutions involve substitutions between amino acids in the same class. Non-conservative substitutions constitute exchanging a member of one of these classes for a member of another.

Percentage sequence identities are determined with antibody sequences maximally aligned by the Kabat numbering convention for a variable region or EU numbering for a constant region. For other proteins, sequence identity can be determined by aligning sequences using algorithms, such as BESTFIT, FASTA, and TFASTA in the Wisconsin Genetics Software Package Release 7.0, Genetics Computer Group, 575 Science Dr., Madison, WI), using default gap parameters, or by inspection, and the best alignment. After alignment, if a subject antibody region (e.g., the entire mature variable region of a heavy or light chain) is being compared with the same region of a reference antibody, the percentage sequence identity between the subject and reference antibody regions is the number of positions occupied by the same amino acid in both the subject and reference antibody region divided by the total number of aligned positions of the two regions, with gaps not counted, multiplied by 100 to convert to percentage.

Compositions or methods "comprising" one or more recited elements can include other elements not specifically recited. For example, a composition that comprises antibody can contain the antibody alone or in combination with other ingredients.

A humanized antibody is a genetically engineered antibody in which the CDRs from a non-human "donor" antibody are grafted into human "acceptor" antibody sequences (see, e.g., Queen, US 5,530,101 and 5,585,089; Winter, US 5,225,539; Carter, US 6,407,213; Adair, US 5,859,205 and 6,881,557; Foote, US 6,881,557). The acceptor antibody sequences can be, for example, a mature human antibody sequence, a composite of such sequences, a consensus sequence of human antibody sequences, or a germline region sequence. Thus, a humanized antibody is an antibody having some or all CDRs entirely or substantially from a donor antibody and variable region framework sequences and constant regions, if present, entirely or substantially from human antibody sequences. Similarly, a humanized heavy chain has at least one, two and usually all three CDRs entirely or substantially from a donor antibody heavy chain, and a heavy chain variable region framework sequence and heavy chain constant region, if present, substantially from human heavy chain variable region framework and constant region sequences. Similarly, a humanized light chain has at least one, two and usually all three CDRs entirely or substantially from a donor antibody light chain, and a light chain variable region framework sequence and light chain constant region, if present, substantially from human light chain variable region framework and constant region sequences. Other than nanobodies and dAbs, a humanized antibody comprises a humanized heavy chain and a humanized light chain. A CDR in a humanized antibody is substantially from a corresponding CDR in a non-human antibody when at least 85%, 90%, 95% or 100% of corresponding residues (as defined by Kabat) are identical between the respective CDRs. The variable region framework sequences of an antibody chain or the constant region of an antibody chain are substantially from a human variable region framework sequence or human constant region, respectively, when at least 85, 90, 95 or 100% of corresponding residues defined by Kabat are identical.

Although humanized antibodies often incorporate all six CDRs (preferably as defined by Kabat) from a mouse antibody, they can also be made with less than all CDRs (e.g., at least 3, 4, or 5 CDRs from a mouse antibody) (e.g., Pascalis et al., J. Immunol. 169:3076, 2002; Vajdos et al., Journal of Molecular Biology, 320: 415-428, 2002; Iwahashi et al., Mol. Immunol. 36:1079-1091, 1999; Tamura et al., Journal of Immunology, 164:1432-1441, 2000).

A chimeric antibody is an antibody in which the mature variable regions of light and heavy chains of a non-human antibody (e.g., a mouse) are combined with human light and heavy chain constant regions. Such antibodies substantially or entirely retain the binding specificity of the mouse antibody, and are about two-thirds human sequence.

A veneered antibody is a type of humanized antibody that retains some and usually all of the CDRs and some of the non-human variable region framework residues of a non-human antibody, but replaces other variable region framework residues that can contribute to B- or T-cell epitopes, for example exposed residues (Padlan, Mol. Immunol. 28:489, 1991) with residues from the corresponding positions of a human antibody sequence. The result is an antibody in which the CDRs are entirely or substantially from a non-human antibody and the variable region frameworks of the non-human antibody are made more human-like by the substitutions.

A human antibody can be isolated from a human, or otherwise result from expression of human immunoglobulin genes (e.g., in a transgenic mouse, in vitro or by phage display). Methods for producing human antibodies include the trioma method of Oestberg et al., Cys muoma 2:361-367 (1983); Oestberg, U.S. Patent No. 4,634,664; and Engleman et al., US Patent 4,634,666, use of transgenic mice including human immunoglobulin genes (see, e.g., The monoclonal antibodies can also be produced by transgenic mice bearing human immune system genes, such as the VelocImmune® mouse from Regeneron Pharmaceuticals, Inc. (Murphy, PNAS 111 no. 14, 5153-5158 (2014)), Xenomouse, Jakobovits, Nature Biotechnology 25, 1134-1143 (2007) or HuMAb mouse from Medarex, Inc. (Lonberg, Handbook Exp. Pharmacol. 181, 69-97 (2008); Lonberg et al., WO93/12227 (1993); US 5,877,397, US 5,874,299, US 5,814,318, US 5,789,650, US 5,770,429, US 5,661,016, US 5,633,425, US 5,625,126, US 5,569,825, US 5,545,806, Nature 148, 1547-1553 (1994), Nature Biotechnology 14, 826 (1996), Kucherlapati, WO 91/10741 (1991). Human antibodies can also be produced by phage display methods (see, e.g., Dower et al., WO 91/17271 and McCafferty et al., WO 92/01047, US 5,877,218, US 5,871,907, US 5,858,657, US 5,837,242, US 5,733,743 and US 5,565,332).

When an antagonist is said to retain a property of a parental antibody from which it was derived, the retention can be complete or partial. Complete retention of an activity means the activity of the antagonist is the same within experimental error or greater than that of the molecule from which it was derived. Partial retention of activity means activity significantly above background level of a negative control (i.e., beyond experimental error) and preferably at least 50% of the corresponding activity of the molecule from which it was derived.

Two antibodies have the same epitope if all amino acid mutations in the antigen that reduce or eliminate binding of one antibody reduce or eliminate binding of the other. Two antibodies have overlapping epitopes if some amino acid mutations that reduce or eliminate binding of one antibody reduce or eliminate binding of the other.

Competition between antibodies is determined by an assay in which an antibody under test inhibits specific binding of a reference antibody to a common antigen (see, e.g., Junghans et al., Cancer Res. 50:1495, 1990). A test antibody competes with a reference antibody if an excess of a test antibody (e.g., at least 2x, 5x, 10x, 20x or 100x) inhibits binding of the reference antibody by at least 50%, but preferably 75%, 90% or 99%, as measured in a competitive binding assay. Antibodies identified by competition assay (competing antibodies) include antibodies binding to the same epitope as the reference antibody and antibodies binding to an adjacent epitope sufficiently proximal to the epitope bound by the reference antibody for steric hindrance to occur.

### DETAILED DESCRIPTION

### I. Overview

The disclosure provides methods for treating Fibrodysplasia Ossificans Progressiva (FOP) in which an effective regime of an antibody against Activin B, BMP9 or BMP10 is administered to a subject having this condition. This disclosure is based in part on the result that these ligands among others can each induce heterotopic ossification of FOP in cells with ACVR1H206 mutation. The activation by Activin B is particularly surprising because Activin B is not a ligand of wild type ACVR1.

Although practice of the invention is not dependent on an understanding of mechanism, a possible explanation of activation of ACVR1H206 but not ACVR1 by Activin B is shown schematically in Figs. 1A-D. Fig. 1A shows Activin B signaling via the type I receptors ACVR1B/1C and Smad2/3 phosphorylation, and sharing type II receptors (ACVR2A, ACVR2B, and BMPR2) with BMPs. Fig. 1B shows ACVR1 together with the type II receptors, recognizing BMPs and stimulates phosphorylation of Smad1/5/8. In Fig. 1C, ACVR1, together with the type II receptors, binds Activin B but the resulting complex does not stimulate phosphorylation of Smad1/5/8; instead, Activin B acts as a competitive inhibitor of canonical BMP-mediated signaling through ACVR1. In Fig. 1D the R206H variant of ACVR1 responds to Activin B, inducing Smad1/5/8 phosphorylation, just like a BMP, effectively converting the ACVR2•ACVR1•Activin complex from a 'dead end' complex into a signaling complex. The R206H variant can also respond to canonical BMPs, such as BMP9 and BMP10. The ACVR2•ACVR1•Activin B complex is shown here as containing a heterodimer of ACVR1-ACVR1[R206H]. However, this is not an obligate arrangement: a homodimer of ACVR1[R206H] is also capable of transducing the signal.

### II. ACVR1, ACVR2A, ACVR2B, Activin A, Activin B, BMP9 and BMP10

The transforming growth factor β (TGFβ) superfamily of ligands includes, for example, bone morphogenetic proteins (BMPs) and growth and differentiation factors (GDFs). The receptors for these ligands are heteromeric receptor complexes made up of type I and type II transmembrane serine/threonine kinase receptors. Examples of type I receptors include activin receptor type IA (ACTRIA, ACVR1, or ALK2), BMP receptor type IA and BMP receptor type IB. Examples of type II receptors include activin receptors type IIA and IIB (ACTRIIA or ACVR2A and ACTRIIB or ACVR2B) and BMP receptor type II. The ligands of the TGFβ superfamily each have differing affinities for the different type I and type II receptors.

Both the type I and type II receptors have an extracellular ligand binding domain (ECD) and an intracellular serine/threonine kinase domain. In addition, the type I receptors have a glycine/serine-rich region (GS-box) preceding the kinase domain and a L45 loop within the kinase domain. Both receptors work together for ligands to activate downstream signaling pathways, such as Smad and non-Smad signaling pathways. Activation involves ligand binding, ligand-receptor oligomerization and transphosphorylation of the GS box of the type I receptor by the type II receptor kinase. The type II receptor kinase is constitutively active and has a role in ligand binding and activation of the type I receptor.

ACVR1, also known as activin a receptor type I, ACVR1A, ACVRLK2, or ALK2, is a type I receptor for the TGFβ superfamily of ligands. ACVR1 has serine/threonine kinase activity and phosphorylates Smad proteins and activates downstream signaling pathways. ACVR1 is found in many tissues of the body including skeletal muscle and cartilage and helps to control the growth and development of the bones and muscles. As described elsewhere herein, certain mutations in the *ACVR1* gene cause FOP. Examples of ACVR1 activity include the ability to bind to ligands, the ability to form a complex with a type II receptor, or the ability to activate downstream signaling pathways, such as the Smad pathway.

ACVR2, also known as activin receptor type II, is a type II receptor for the TGFβ superfamily of ligands. There are at least two ACVR2 receptors, for example, activin receptor type IIA (ACVR2A or ACTRIIA) and activin receptor type IIB (ACVR2B or ACTRIIB). Reference to ACVR2 includes either or both of ACVR2A and ACVR2B. ACVR2A and ACVR2B can be expressed in multiple tissues, including skeletal muscle, stomach, heart, endometrium, testes, prostate, ovary, and neural tissues.

On ligand binding, an ACVR2 receptor forms a complex with a type I receptor, such as ACVR1, and phosphorylates the GS box of the type I receptor, thus enhancing the kinase activity of the type I receptor. Examples of ACVR2A and ACVR2B activity include the ability to bind to ligands, the ability to form a complex with a type I receptor, or the ability to phosphorylate a type I receptor.

An exemplary form of human ACVR2A has Swiss Prot accession number P27037. Residues 1-19 are a signal peptide, residues 20-135 are an extracellular domain, residues 59-116 are an activin types I and II receptor domain, residues 136-161 are a transmembrane domain and residues 162-513 are a cytoplasmic domain. An exemplary form of human ACVR2B is assigned Swiss Prot Number Q13705. Residues 1-18 are a signal sequence, residues 19-137 are an extracellular domain, residues 27-117 are an activin types I and II receptor domain, residues 138-158 are a transmembrane domain and residues 159-512 are a cytoplasmic domain. An exemplary form of human ACVR1 has Swiss Prot accession number Q04771. Residues 1-20 are a signal sequence, residues 21-123 are extracellular domain, residues 33-104 are an activin types I and II receptor domain, residues 124-146 are a transmembrane domain and residues 147-509 are a cytoplasmic domain. Reference to any of ACVR1, ACVR2A and ACVR2B includes these exemplary forms, known isoforms and polymorphisms thereof, such as those listed in the Swiss Prot database, cognate forms from other species, and other variants having at least 90, 95, 96, 97, 98 or 99% sequence identity with an exemplified form.

Residues of forms of ACVR2A, ACVR2B and ACVR1 other than the exemplified sequences defined above are numbered by maximum alignment with the corresponding exemplified sequences so aligned residues are allocated the same number.

Activin A in humans can exist as a homo or heterodimeric protein. The homodimeric protein contains a homodimeric beta A subunit pair. The heterodimeric protein contains a beta A subunit and a beta B, beta C or beta E subunit (i.e., beta A beta B, beta A beta C, or beta A beta E). The subunits are each expressed as precursor polypeptides including a signal peptide, propeptide and mature polypeptide. An exemplary form of human beta A subunit precursor is a polypeptide of length 426 amino acids designated Swiss Prot P08476 of which residues 1-20 are a signal peptide, residues 21-310 are a propeptide and residues 311-426 are the mature polypeptide. An exemplary form of a beta B subunit precursor polypeptide is designated Swiss Prot P09529 of which residues 1-28 are a signal peptide, residues 29-292 a propeptide and residues 293-407 a mature polypeptide. An exemplary form of a beta C subunit is designated Swiss Prot P55103, of which residues 1-18 are a signal peptide, residues 19-236 are a propeptide and residues 237-352 are a mature polypeptide. An exemplary form of a beta E subunit precursor is designated Swiss Prot P58166 of which residues 1-19 are a signal peptide, residues 20-236 are a propeptide and residues 237-350 are a mature polypeptide. Several variants of these sequences are known as described in the Swiss Prot Database. Reference to Activin A includes any of the beta A homodimer, beta A beta B, beta A beta C and beta A beta E heterodimer forms, as well as their subunits, as well as their precursors in which subunits are attached to the propeptide and/or signal peptide defined by the exemplary Swiss Prot sequences provided or other natural occurring human forms of these sequences. Activin A signals through binding to ACVR2A or ACVR2B, but is not known to be a ligand for ACVR1.

Activin B exists as a homodimer of beta B subunits. Activin B signals through binding to ACVR2A and ACVR2B but is not known to be a ligand of ACVRI. Reference to Activin B refers to the homodimer, or a beta B subunit polypeptide, which can be a full length beta B polypeptide or the mature polypeptide portion thereof free of peptide and propeptides, defined by the exemplary Swiss Prot sequence provided or other natural occurring human forms of this sequence.

An exemplary form of human BMP9 has been assigned Swiss Prot Q9UK05. This protein has 429 amino acids of which residues 1-22 are a signal peptide, residues 23-319 are a propeptide and residues 320-429 are a mature polypeptide. BMP9 naturally exists as a disulfide-bonded homodimer. It is thought to interact with ACVRI. Reference to BMP9 refers to the homodimer or a subunit thereof, which can be a full length BMP9 polypeptide or mature polypeptide portion thereof free of the signal peptide and propeptide defined by the exemplary Swiss Prot sequence provided or other natural occurring human forms of this sequence.

An exemplary form of human BMP10 has been assigned Swiss-Prot O95393, of which residues 1-21 are a signal peptide, residues 22-316 are a pro-peptide and residues 317-424 are the mature peptide. BMP10 exists as disulfide bonded homodimer. It is thought to interact with ACVRI. Reference to BMP10 refers to the homodimer or subunit thereof, which can be a full length BMP10 polypeptide or a mature polypeptide portion thereof free of the signal peptide and propeptide, defined by the exemplary Swiss Prot sequences provided or other natural occurring human forms of this sequence.

### III. Antibodies against Activin B, BMP9 and BMP10

Each of Activin B, BMP9 and BMP10 is a naturally found as a homodimer. Antibodies against one of these targets can specifically bind to the homodimer without binding to the monomeric subunit from which the homodimer is formed (i.e., both paired subunits contribute to the epitope), or can specifically bind to both homodimer and a monomeric subunit, or can specifically bind to the monomeric subunit without binding to the homodimer (epitope within subunit obscured when it is associated with another subunit). Some antibodies against Activin B also specifically bind to Activin AB (epitope within the beta B subunit), whereas other antibodies specifically bind to Activin B without binding to Activin AB (both beta B subunits contribute to the epitope). Some antibodies against Activin B specifically bind to inhibin B, whereas others do not. Some antibodies against BMP9 or BMP10 specifically bind to BMP9 without specifically binding to BMP10 or vice versa. Some antibodies specifically bind to both BMP9 and BMP10. Unless otherwise specified, antibodies specifically bind to the human form of their target. Antibodies may or may not also bind to non-human cognate forms of their target. For testing in non-human cells, each mouse cells, it is preferred that an antibody cross react with its human target and the cognate form of that target in the species being tested.

Preferred antibodies inhibit signaling of their target ligand through ACVR1H206 in an assay described in Example 1. Some antibodies inhibit signal transduction of their target ligand with an IC50 of less than 4 nM, and sometimes less than 400 pM or 40 pM. Some antibodies inhibit signal transduction with and IC50 in a range of 4 nM to 10 pM or 3.5 nM to 35 pM. Preferred antibodies inhibit heterotopic ossification symptoms of FOP in an animal model as described in Example 2.

Several monoclonal antibodies against BMP9 are commercially available or described in scientific or patent literature. These include MAB3209 of US20140227254 available from R & D Systems, Inc., 4D2 from Novus Biologicals, LLC, and antibodies 6D10-1-1, 10D5-2-3 and 3B7-3-3 as described in US20140056902.

Commercially available monoclonal antibodies against BMP10 include MAC106Hu22 (USCN Life Science Inc.) MM0113-5L26 Ab CAM PLC, and MAB 2926 R & D Systems, Inc.

Monoclonal antibodies against Activin B that are commercially available or described in the patent literature include clone 146807 from Sigma Aldridge or R & S Biosystems, Inc., 9M29 from GeneTex, Inc. and 46A/F of US20090317921.

Humanized, chimeric and veneered forms of any of these antibodies are included as are antibodies competing for binding therewith or sharing the same epitope. Other antibodies can be obtained by mutagenesis of cDNA encoding the heavy and light chains of any of the above-mentioned antibodies. Monoclonal antibodies that are at least 90%, 95% or 99% identical to any of the above-mentioned antibodies in amino acid sequence of the mature heavy and/or light chain variable regions and maintain its functional properties, and/or which differ from the respective antibody by a small number of functionally inconsequential amino acid substitutions (e.g., conservative substitutions), deletions, or insertions are also included in the invention. Monoclonal antibodies having at least 1, 2, 3, 4, 5 and preferably all six CDR(s) that are 90%, 95%, 99% or 100% identical to corresponding CDRs of any of the exemplified antibodies are also included. CDRs are preferably as defined by Kabat, but can be defined by any conventional alternative definition, such as Chothia, composite Kabat-Chothia, the contact definition or AbM definition (see world wide web bioinf.org.uk/abs).

Specific binding of an antibody or fusion protein to its target antigen means an affinity of at least 10⁶, 10⁷, 10⁸, 10⁹, or 10¹⁰ M⁻¹. Specific binding is detectably higher in magnitude and distinguishable from non-specific binding occurring to at least one unrelated target.

Reference to an antibody includes intact antibodies with two pairs of heavy and light chains, and antibody fragments that can bind antigen (*e.g.,* Fab, F(ab')₂, Fv, single chain antibodies, diabodies, antibody chimeras, hybrid antibodies, bispecific antibodies, humanized antibodies, and the like), and recombinant peptides comprising the foregoing. Antibody fragment refer to fragments including an antigen-binding portion of an intact antibody. Examples of antibody fragments include Fab, F(ab')2, and Fv fragments; diabodies; linear antibodies (Zapata et al. (1995) Protein Eng. 10:1057-1062); single-chain antibody molecules; and multispecific antibodies formed from antibody fragments.

The antibody can be monoclonal or polyclonal. A monoclonal antibody is an antibody obtained from a population of substantially homogeneous antibodies, that is, the individual antibodies comprising the population are identical except for possible naturally occurring mutations that can be present in minor amounts. Monoclonal antibodies are often highly specific, being directed against a single antigenic site. Furthermore, in contrast to conventional (polyclonal) antibody preparations that typically include different antibodies directed against different determinants (epitopes), each monoclonal antibody is typically directed against a single determinant on the antigen. The modifier "monoclonal" indicates the character of the antibody as being obtained from a substantially homogeneous population of antibodies, such as those produced by a clonal population of B-cells, and does not require production of the antibody by any particular method.

Monoclonal antibodies can be made by the hybridoma method first described by Kohler et al. (1975) Nature 256:495, or a modification thereof. Typically, an animal, such as a mouse, is immunized with a solution containing an antigen (e.g. an Activin B, BMP9 or BMP10 homodimer or subunit thereof or portion thereof).

Immunization can be performed by mixing or emulsifying the antigen-containing solution in saline, preferably in an adjuvant such as Freund's complete adjuvant, and injecting the mixture or emulsion parenterally. After immunization of the animal, the spleen (and optionally, several large lymph nodes) are removed and dissociated into single cells. The spleen cells can be screened by applying a cell suspension to a plate or well coated with the antigen of interest. The B-cells expressing membrane bound immunoglobulin specific for the antigen bind to the plate and are not rinsed away. Resulting B-cells, or all dissociated spleen cells, are then induced to fuse with myeloma cells to form hybridomas, and are cultured in a selective medium. The resulting cells are plated by serial dilution and are assayed for the production of antibodies that specifically bind the antigen of interest (and that do not bind to unrelated antigens). The selected monoclonal antibody (mAb)-secreting hybridomas are then cultured either *in vitro* (e.g., in tissue culture bottles or hollow fiber reactors), or *in vivo* (as ascites in mice).

Alternatively, the monoclonal antibodies can be made by recombinant DNA methods (see, *e.g.,* U.S. Patent No. 4,816,567). The monoclonal antibodies can also be isolated from phage antibody libraries using the techniques described in, for example, Clackson et al. (1991) Nature 352:624-628; Marks et al. (1991) J. Mol. Biol. 222:581-597; and U.S. Patent No. 5,514,548.

The present monoclonal antibodies can be any of the various antibody isotypes, namely IgG, IgM, IgE, IgD, or IgA class. Monoclonal antibodies of isotype IgG are preferred. The isotype can be any of IgG1, IgG2, IgG3 or IgG4, particularly human IgG1, IgG2, IgG3 or IgG4. Human IgG1 is often preferred if effector functions are desired and IgG2 or IgG4 if they are not, although alternatively human IgG1 can be mutated to attenuated effector functions if not desired.

One or several amino acids at the amino or carboxy terminus of the light and/or heavy chain, such as a C-terminal lysine of the heavy chain, can be missing or derivatized in a proportion or all of the molecules. Substitutions can be made in the constant regions to reduce or increase effector function such as complement-mediated cytotoxicity or ADCC (see, e.g., Winter et al., US Patent No. 5,624,821; Tso et al., US Patent No. 5,834,597; and Lazar et al., Proc. Natl. Acad. Sci. USA 103:4005, 2006), or to prolong half-life in humans (see, e.g., Hinton et al., J. Biol. Chem. 279:6213, 2004). Exemplary substitutions include a Gln at position 250 and/or a Leu at position 428 (EU numbering) for increasing the half-life of an antibody. Substitution at any of positions 234, 235, 236 and/or 237 reduces affinity for Fcγ receptors, particularly FcγRI receptor (see, e.g., US 6,624,821). Optionally, positions 234, 236 and/or 237 in human IgG2 are substituted with alanine and position 235 with glutamine. (See, e.g., US 5,624,821). Effector functions can also be reduced by substitution of EFLG at positions 232-236 with PVA (see WO14/121087). Optionally, S at position 428 can be replaced by P, particularly in human IgG4 to reduce exchange between endogenous and exogenous immunoglobulins. Other variations can add or remove sites of post-translational modification, such as N-linked glycosylation at N-X-S/T motifs. Variations can also include introduction of knobs (i.e., replacement of one or more amino acids with larger amino acids) or holes (i.e., replacement of one or more amino acids with smaller amino acids) to promote formation of heterodimers between different heavy chains for production of bispecific antibodies. Exemplary substitutions to form a knob and hole pair are T336Y and Y407T, respectively (Ridgeway et al., Protein Engineering vol.9 no.7 pp.617-621, 1996). Variations can also include mutations that reduce protein A interaction (e.g., H435R and Y436F) in the EU numbering system. Bispecific antibodies in which one heavy chain has such a variation, and another does not, can be separated from their parental antibodies by protein-A affinity chromatography.

### IV. Fibrodysplasia Ossificans Progressiva (FOP)

FOP is a rare heritable disorder in which heterotopic ossification forms histologically and biomechanically 'normal' bone at extraskeletal sites, such as connective tissue. This disorder, although episodic, is cumulative, and results in permanent disability of increasing severity.

FOP's worldwide prevalence is approximately 1/2,000,000. There is no ethnic, racial, gender, or geographic predilection to FOP. It is not only an extremely disabling disease but also a condition of considerably shortened lifespan.

Characteristics of FOP include, for example, congenital malformations of the great toe, flare-ups characterized by painful soft tissue swellings on the head, neck, and/or back with inflammation and progressive formation of heterotopic bone via endochondral ossification.

FOP can be suspected clinically based on the presence of malformations of the great toe. Diagnostic tests, such as x-rays or bone scan can substantiate great toe abnormalities and confirm the presence of heterotopic ossification. A FOP diagnosis can also be confirmed by genetic testing, for example, by detecting the 617 G-to-A (R206H) mutation in the *ACVR1* gene.

It is common for FOP to be misdiagnosed as several other disorders, including other conditions of heterotopic ossification. FOP should be distinguished by a differential diagnosis from disorders including, for example, isolated congenital malformations, lymphedema, soft tissue sarcoma, desmoid tumors, aggressive juvenile fibromatosis, juvenile bunions, isolated brachydactyly, progressive osseous heteroplasia and heterotopic ossification. The presence of great toe congenital malformations and the painful soft-tissue flare-ups can be used to differentiate FOP from other disorders.

Patients with FOP have congenital malformations of the great toe but otherwise appear normal at birth. The flare-ups associated with FOP start during the first decade of life. Flare-ups can be triggered by, for example, soft tissue injury, falls, fatigue, viral infections or intramuscular injections. The result of the flare-ups is a transformation of soft tissue, such as ligaments, skeletal muscle or tendons into heterotopic bone.

There was no previous therapeutic treatment for FOP. FOP was managed by preventative measures, such as improved safety and strategies to minimize injury, avoiding intramuscular injections and taking care when receiving dental care. High dose corticosteroid treatments started within the first 24 hours of a flare-up can help reduce the inflammation and edema associated with flare-ups. Surgical strategies to remove the heterotopic bone are not recommended as it is counterproductive and causes new trauma-induced heterotopic ossification.

FOP is caused by mutations in *ACVR1* (also known as *ALK2*) that appear to destabilize the interaction of the GS domain with an inhibitory molecule, FKBP12 (Groppe, J., et al. 2011, Cells Tissues Organs, 194:291-295). FKBP12 is a negative modulator of ACVR1 and functions to stabilize the receptor in an inactive conformation (Huse, M., et al. 1999, Cell, 96:425-436). See Kaplan, F.S., et al. 2012, Disease Models & Mechanisms, 5:756-762). An example of a mutation in ACVR1 that is associated with FOP is an Argininc 206 to Histidinc (R206H) mutation in the intracellular domain.

A subject at risk of developing FOP includes any subject with the ACVR1 R206H mutation or other mutation associated with FOP, a subject born with malformations of the great toe, or a subject that has a family history of FOP, who has not yet developed symptoms of FOP sufficient for a diagnosis of FOP to be made by art-recognized criteria.

### V. Methods of Treatment

Disclosed herein are methods of treating FOP, comprising administering to a subject having FOP an effective regime of an antibody against Activin B, BMP9 or BMP10.

A "subject" is any animal (i.e. mammals) such as, humans, primates, rodents, such as mice and rats, agricultural and domesticated animals such as, dogs, cats, cattle, horses, pigs, sheep, and the like, in which one desires to treat FOP. In any of the present methods, the subject can be mammal and preferably human.

An effective regime of an antibody against Activin B, BMP9 or BMP10 means a combination of dose, frequency and route of administration of an antagonist which brings a positive response in at least one sign or symptom of FOP. A positive response can include reducing, eliminating, ameliorating, inhibiting worsening of, or delaying at least one sign or symptom of FOP. Signs or symptoms of FOP that can be subject of a positive response include for example, ectopic or heterotopic bone formation, FOP flare-ups, or pain and swelling associated with flare-ups. The regime can be assessed in a single patient by comparing signs and symptoms before and after treatment. A regime is considered effective if at least one sign or symptom gives a positive response following treatment. A regime can alternatively or additionally be assessed by comparing signs and symptoms of population of subjects treated with an antagonist or antagonists of the present invention with a control population of subjects not receiving treatment. The subjects for such comparison can be an animal model, or human subjects in a clinical trial (e.g., phase I, phase II, IIa, IIb, or III). A regime is considered effective if there is a statistically significant positive response between the populations in at least one sign or symptom.

In some methods for treating FOP, the subject does not have and is not at risk of other conditions treatable with antibody against Activin B, BMP9 or BMP10. For example, the subject can be free of any or all of type II diabetes, muscular dystrophy, amyotrophic lateral sclerosis (ALS) and osteoporosis.

### A. Methods of Administration

An antibody against Activin B, BMP9 or BMP10 is usually administered directly as proteins or small molecules, but in the case of proteins can also be administered as nucleic acid encoding such proteins. Such antagonists can be administered by various methods, such as cellular transfection, gene therapy, direct administration with a delivery vehicle or pharmaceutically acceptable carrier.

Various delivery systems can be used to administer the antibody against Activin B, BMP9 or BMP10, provided herein, e.g., encapsulation in liposomes, microparticles, microcapsules, recombinant cells capable of expressing the compound, receptor-mediated endocytosis (see, e.g., Wu and Wu, 1987, J. Biol. Chem. 262:4429-4432), construction of a nucleic acid as part of a retroviral or other vector, etc.

Methods of administration can be enteral or parenteral and include intradermal, intramuscular, intraperitoneal, intravenous, subcutaneous, pulmonary, intranasal, intraocular, epidural, and oral routes. The compounds can be administered by any convenient route, for example by infusion or bolus injection, by absorption through epithelial or mucocutaneous linings (e.g., oral mucosa, rectal and intestinal mucosa, etc.) and can be administered together with other biologically active agents. Administration can be systemic or local. In addition, it can be desirable to introduce the pharmaceutical compositions of the invention into the central nervous system by any suitable route, including intraventricular and intrathecal injection; intraventricular injection can be facilitated by an intraventricular catheter, for example, attached to a reservoir, such as an Omcana reservoir. Pulmonary administration can also be employed, e.g., by use of an inhaler or nebulizer, and formulation with an aerosolizing agent.

The pharmaceutical compositions disclosed herein can be administered locally to the area in need of treatment; this can be achieved, for example, by local infusion during surgery, topical application, e.g., by injection, by means of a catheter, or by means of an implant, said implant being of a porous, non-porous, or gelatinous material, including membranes, such as sialastic membranes, fibers, or commercial skin substitutes.

Pharmaceutical compositions can also be delivered in a vesicle, in particular a liposome (see Langer (1990) Science 249:1527-1533). Pharmaceutical compositions can also be in a controlled release system, a pump (see Langer (1990) *supra* or with polymeric materials (see Howard et al. (1989) J. Neurosurg. 71:105).

### B. Combination Therapies

An antibody against Activin B, BMP9 or BMP10 can be administered as a monotherapy, or as a combination therapy. For example, antibodies against two or all three of Activin B, BMP9 or BMP10 can be administered in combination therapy. An antibody against any or all of Activin B, BMP9 or BMP10 can also be administered in combination with any or all of an ACVR1 antagonist, an ACVR2A antagonist or an ACVR2B antagonist, or an antibody against Activin A, as further described in US 62/141,775 filed April 1, 2015. Preferred ACVR1, ACVR2A and ACVR2B antagonists are Fc fusion proteins including the extracellular domain linked to an Fc domain. Another preferred antagonist of ACVR1 is the small molecule inhibitor LDN-212854 described by Mohedas et al., (2013) ACS Chem. Biol. 8:1291-1302. Preferred antibodies against Activin A include H4H10446P and H4H10430P in US2015037339 and A1 as described in US 8,309,082. Multiple agents in a combination therapy can be administered simultaneously, or sequentially.

### C. Pharmaceutical Compositions

Disclosed herein are pharmaceutical compositions comprising an antibody against Activin B, BMP9 or BMP10 and a pharmaceutically acceptable carrier. Pharmaceutically acceptable means approved or approvable by a regulatory agency of the Federal or a state government or listed in the US Pharmacopeia or other generally recognized pharmacopeia for use in animals, and more particularly in humans. A carrier is a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Suitable pharmaceutical excipients include starch, glucose, lactose, sucrose, gelatin, malt, rice, flour, chalk, silica gel, sodium stearate, glycerol monostearate, talc, sodium chloridc, dried skim milk, glycerol, propylene, glycol, water, cthanol and the like. The composition, if desired, can also contain minor amounts of wetting or emulsifying agents, or pH buffering agents. Pharmaceutical compositions for parenteral administration are often sterile and substantially isotonic (osmolality of about 250-350 mOsm/kg water) and manufactured under GMP conditions. Pharmaceutical compositions can be provided in unit dosage form (i.e., the dosage for a single administration).

These compositions can take the form of solutions, suspensions, emulsion, tablets, pills, capsules, powders, sustained-release formulations, lyophilisates and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin.

The compositions can be formulated adapted for intravenous or subcutaneous administration to human beings. When necessary, the composition can also include a solubilizing agent and a local anesthetic such as lidocaine to ease pain at the site of the injection. When the composition is to be administered by infusion, it can be dispensed with an infusion bottle containing sterile pharmaceutical grade water or saline. When the composition is administered by injection, an ampoule of sterile water for injection or saline can be provided so that the ingredients can be mixed prior to administration.

The antibody against Activin B, BMP9 or BMP10, disclosed herein can be formulated as neutral or salt forms. Pharmaceutically acceptable salts include those formed with free amino groups such as those derived from hydrochloric, phosphoric, acetic, oxalic, tartaric acids, and the like, and those formed with free carboxyl groups such as those derived from sodium, potassium, ammonium, calcium, ferric hydroxides, isopropylamine, triethylamine, 2-ethylamino ethanol, histidine, procaine, and the like.

The amount and frequency of the antibody against Activin B, BMP9 or BMP10, administered by a specified route effective in the treatment of FOP (e.g. effective regime) can be determined by standard clinical techniques based on the present description. In addition, *in vitro* assays or animal models can be employed to help identify optimal dosage ranges. The precise dose to be employed in the formulation also depends on the route of administration, and the seriousness of the condition, and should be decided according to the judgment of the practitioner and each subject's circumstances. However, suitable dosage ranges for administration are generally about 20-50000 micrograms of active compound per kilogram body weight.

If different versions of a sequence are associated with an accession number at different times, the version associated with the accession number at the effective filing date of this application is meant. The effective filing date means the earlier of the actual filing date or filing date of a priority application referring to the accession number if applicable. Likewise if different versions of a publication, website or the like are published at different times, the version most recently published at the effective filing date of the application is meant unless otherwise indicated. Any feature, step, element, embodiment, or aspect of the invention can be used in combination with any other unless specifically indicated otherwise.

### EXAMPLES

### Example 1: This example identifies ligands inducing heterotopic ossification in cells with an AVCR1[R206H] mutation.

### Materials and Methods

HEK293 cells were transfected with a modified pFA vector (Stratagene) harboring a 10 tandem repeats of a GC-rich BMP Responsive Element (BRE; 5'-GCCGCCGCagc-3') and a 168bp minimal promoter from mouse Osteocalcin gene. BRE was used to drive firefly luciferase expression. The corresponding stable cell line was created with Lipofectamine 2000 (Invitrogen) transfection followed by selection with G418 at 500ug/ml. Unless otherwise noted, cells were cultured in DMEM containing 10 % (v/v) fetal bovine serum, 50 units/ml penicillin/streptomycin and 2 mM L-glutamine. cDNA for human ACVR1 was cloned into the pCMV expression vector (pCMV.ACVR1). The ACVR1[R206H] variant was introduced into pCMV.ACVR1 and confirmed by sequencing. To generate ACVR1 wild type and R206H over-expressing HEK293/BRE-Luc stable cell lines, HEK293/BRE-Luc cells were transfected with pCMV.ACVR1 or pCMV.ACVR1[R206H] using TransIT-LT1 transfection reagent (Mirus) according to the manufacturer's instructions. Stable lines were generated by selection with 100ug/ml hygromycin B. Pools of stably transfected cells expressing nearly identical levels of ACVR1 and ACVR1[R206H] were generated by fluorescence-activated cell sorting (FACS) after surface staining with an anti-ACVR1 antibody (see below). The resulting pools were used for signaling assays.

### FACS-sorting to select ACWR1-expressing cells

For live cell staining, 15 × 10⁶ cells were treated with TrypLE Express Enzyme (Life Technology). Harvested cells were washed with PBS containing 2 % FBS and stained with 5 µg/ml of monoclonal mouse anti human ACVR1 antibody (R&D Systems MAB637) for one-hour at 4°C. Cells were then washed once with PBS containing 2 % FBS and goat anti-mouse IgG Alexa Flour 647 (Invitrogen) was used for detection. After two washes in PBS containing 2 % FBS, cells were filtered with 70 µm cell strainer. DAPI-positive, live cells were sorted for surface expression of hACVR1. Pools were collected with MoFlo XDP (Beckman Coulter). Expression of ACVR1 was confirmed after FACS-based cell sorting by an additional round of surface staining.

### Signaling assays in HEK293/Bre-Luc reporter lines

HEK293/BRE-Luc reporter cell lines were plated in 96-well plate (Thermo Scientific) at 10,000 cells/0.33 cm² and were incubated at 37°C for 3-hour. Cells were treated as described. For competition assays, rhActivin A and rhBMP6 (R&D Systems) were pre-mixed at indicated concentrations before addition to cells. For experiments that included Activin A antibody, reporter cells were plated as above and Activin A antibody was pre-incubated for 30 minutes with recombinant Activins before addition to cells. For all reporter assays, luciferase expression was measured 16 hours after treatments with Bright-Glo Luciferase Assay System (Promega).

### Results:

HEK293/BRE-Luc reporter lines stably expressing nearly identical levels of either ACVR1 or ACVR1[R206H] were tested for their response to a panel of ligands belonging to the BMP and TGFβ families. ACVR1[R206H] displayed increased signaling in response to some, but not all, of its canonical ligands. Specifically, the response to BMP2, BMP4, BMP7, BMP9, and BMP10 was enhanced, whereas the response to BMP2/7, BMP4/7, BMP5, and BMP6 was unchanged (*Table 1*)*.* These experiments also uncovered an unexpected property of ACVR1[R206H]; that it has become responsive to Activin A, AB, AC, Activin B, and perhaps BMP15, a set of 'non-canonical' ligands to which wild type ACVR1 shows no measurable response.

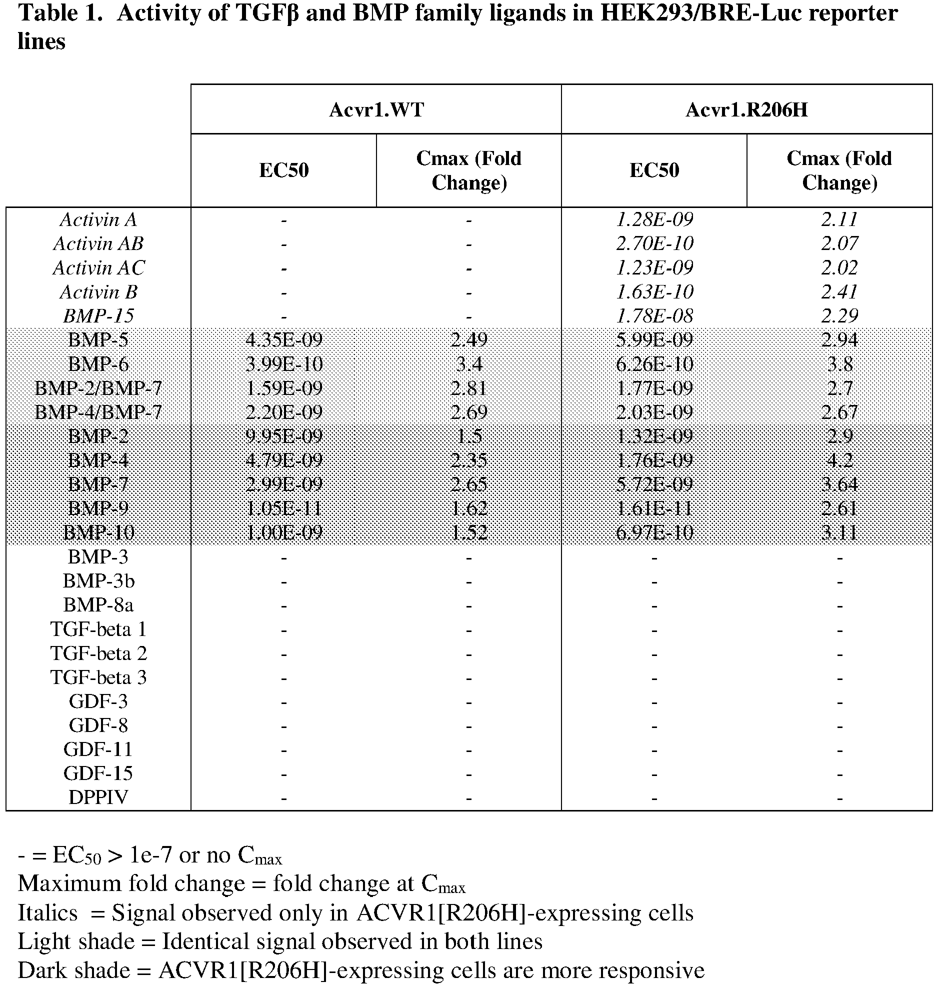

To exclude the possibility that the observed results were an artifact of overexpression, we examined whether the altered signaling properties of ACVR1[R206H] would reproduce in the knock-in *Acvr1*^{*[R206H]FlEx*/+} *; Gt(ROSA26)Sor*^{*CreERT2*/+} ES cells. During the course of these experiment we discovered that prior to activation by Cre, the *Acvr1^{[R206H]FlEx}* allele is hypomorphic with about half of the transcripts missing exon 5. Therefore, from a functional standpoint, *Acvrl1*^{*[R206H]FlEx*/+} cells are nearly equivalent to *Acvr1*^{*null*/+}*.* Nonetheless, in agreement with the data obtained in HEK293 cells, comparison between *Acvr1*^{*[R206H]FlEx*/+} *; Gt(ROSA26)Sor*^{*CreERT2*/+} ES cells and their 'Cre-activated' counterpart, *Acvr1*^{*[R206H]*/+} ; *Gt(ROSA26)Sor*^{*CreERT2*/+} ES cells, showed that the latter have become responsive to Activin A. This altered responsiveness is also observed in *Acvr1*^{*[R206H]*/}*^{null} ; Gt(ROSA26)Sor*^{*CreERT2*/+} ES cells, indicating that one allele of Acvr1[R206H] is sufficient for altered signaling, and that presence of a wild type allele does not significantly modify this aberrant response, though it may contribute to increase the signal. Activation of signaling via Activin A•Acvr1[R206H] utilized Smad1/5/8 and did not involve conversion of signaling to Smad2/3. Furthermore, the presence of the Acvr1[R206H] variant in these cells does not appear to significantly alter Activin A signaling via its canonical receptor and Smad2/3.

These data indicates that an amino acid change in the intracellular domain of a type I BMP receptor is capable of changing the responsiveness of that receptor in a qualitative way, rendering it permissive to activation by non-canonical ligands. One possible explanation of how this happens might lie in the reduced affinity that ACVR1[R206H] displays for FKBP12 (Groppe et al., Clin Orthop Relat Res 462, 87 (Sep, 2007) and Cells, Tissues, Organs 194, 291 (2011). Therefore, we tested whether wild type ACVR1 could be converted into an Activin-responsive receptor if FKBP12 binding was reduced by FK506. We show that the only effect of FK506 is to enhance signaling from canonical ligands - it does not enable wild type ACVR1 to respond to Activin A.

We also examined whether the responsiveness to non-canonical ligands could be simply explained by ACVR1[R206H] gaining the ability to bind them. This was tested in assays that utilize artificial fusion receptors that report association between type I and type II BMP receptors by LacZ complementation (DiscovRx, Fremont, CA). We demonstrate that ACVR1 forms heterodimers with ACVR2A in the presence of these non-canonical ligands. These data indicate that the altered responsiveness of ACVR1[R206H] cannot be attributed to newly acquired binding properties by this receptor. Furthermore, the ability of Activins to form non-signaling complexes with ACVR1 and ACVR2 provides evidence that Activins can act as competitive inhibitors of canonical BMP signaling through ACVR1. Indeed, Activin A inhibits BMP6-induced signaling in a competition assay utilizing HEK293/BRE-Luc reporter cells overexpressing wild type ACVR1 (*Fig. 2*)*.* Taken together, these results indicate that the most striking and unique differentiating property of ACVR1[R206H] over wild type ACVR1 is the former's ability to transduce signal from non-canonical ligands. In addition, they provide evidence that non-canonical ligands may at times function as competitive inhibitors of canonical ACVR1-mediated signaling.

### Example 2: Use of an antibody against Activin B, BMP9 or BMP10 to suppress ectopic bone formation in a mouse model of FOP.

Transgenic knock-in mice have been developed that carry a conditional allele encoding Acvr1[R206H]. These *Acvr1*^{*[R206H]COIN*/+} mice are described in US 14/207,320 and PCT/US2014/026582. This allele expresses the R206H variant only after activation by Cre recombinase. This allows Cre-dependent activation of Acvr1[R206H] expression at specific tissues and at specific time by using different types of Cre driver lines. In this manner the resulting mice also bypass the perinatal lethality that has been observed with a non-regulated knock-in allele of Acvr1[R206H]. Activation of Acvr1[R206H] expression in young or in adult mice results in ectopic bone formation. For example, *Acvr1*^{*[R206II]COIN*/+}; *Gt(ROSA26)Sor*^{*CreERt2*/+} mice (wherein CreERt2 is a tamoxifen-regulatable recombinase (see Feil et al. (1997) Biochem. Biophys. Res. Commun. 237(3):752-7) that has been introduced into the Gt(ROSA26)Sor locus, and hence it is constitutively and globally expressed) develop FOP after exposure to tamoxifen. Briefly, in the absence of tamoxifen, CreERt2 is inactive. Tamoxifen activates expression of Cre which then acts upon the *Acvr1*^{*[R206H]COIN*/+} to convert it to *Acvr1*^{*[R206H]*/+}*,* thereby converting the genotype of the mice to mirror the genotype of the FOP patients that are ACVR1[R206H]. The Acvr1[R206H] allele expresses Acvr1[R206H], and that is adequate to drive the development of FOP in the *Acvr1*^{*[R206H]*/+}; *Gt(ROSA26)Sor*^{*CreERt2*/+} mice. This bypasses the embryonic lethality experienced with conventional Acvr1[R206H] knock-in mice, Acvr1tm1Emsh (http://www.informatics.jax.org/allele/key/828153).

*Acvr1*^{*[R206H]COIN*/+}; *Gt(ROSA26)Sor*^{*CreERt2*/+} mice are given tamoxifen at 1mg/mouse dose i.p. for eight days. Mice are treated with 10mg/kg of antibody against Activin B, BMP9 or BMP10 or 10mg/kg irrelevant control antibody twice weekly for 6 weeks. Mice are monitored using in vivo µCT at baseline, 2, 4 and 6 weeks post initiation of tamoxifen administration.

Figs. 3A-G show heterotopic bone formation in the *Acvr1*^{*[R206H]FlEx*/+} ; *Gt(ROSA26)Sor*^{*CreERT2*/+} mice without treatment with an antibody against Activin B, BMP9 or BMP10 6 weeks after tamoxifen administration. Ectopic bone growth was found at a number of different sites including adjacent to the existing skeleton in the (A) sternum, (B) caudal vertebrae and (C) hip joint. (D) Ectopic bone growth formed between 2 and 4 weeks after tamoxifen injection and can occur distal to the existing skeleton. These ectopic bone lesions can fuse with the existing skeleton. (E) Ex-vivo µCT image of an ectopic bone lesion from the dorsal view showing bridging from the femur to the pelvis. (F) A transverse view through the ectopic bone shows that the newly formed bone has both cortical and trabecular like structures. At the region of intersection of the ectopic bone and the endogenous skeleton (arrowhead) there is evidence of remodeling of the cortical bone, but no evidence of bone marrow sharing. (G) H&E stained histological sections of ectopic bone lesion demonstrates cortical (c) and trabecular bone (t) like structures and bone marrow (bm).

After 6 weeks, more mice in the control group than the treated group develop ectopic bone in at least one location.

## Claims

1. An antibody against Activin B for use in a method of treating Fibrodysplasia Ossificans Progressiva (FOP), wherein the antibody against Activin B is an antagonist antibody.

2. The antibody for use according to claim 1, wherein the antibody is chimeric, veneered, humanized or human antibody.

3. The antibody for use according to any of the preceding claims, wherein the antibody is an intact antibody with two pairs of heavy and light chains.

4. The antibody for use according to any of the preceding claims, wherein the antibody is a human kappa IgG1 antibody.

5. The antibody for use according to any of the preceding claims, wherein the use comprises administering the antibody in combination with an antibody against BMP9 or BMP10.

6. The antibody for use according to any one of claims 1-4, wherein the use comprises administration of the antibody in combination therapy with an ACVR1, ACVR2A, or ACVR2B extracellular domain-Fc fusion protein or an antibody against Activin A.

## Patentansprüche

1. Antikörper gegen Activin B zur Verwendung in einem Verfahren zur Behandlung von Fibrodysplasia ossificans progressiva (FOP), wobei der Antikörper gegen Activin B ein Antagonisten-Antikörper ist.

2. Antikörper zur Verwendung nach Anspruch 1, wobei der Antikörper ein chimärer Antikörper ist, ein Antikörper, der Veneering unterzogen wurde, ein humanisierter oder ein menschlicher Antikörper ist.

3. Antikörper zur Verwendung nach einem der vorangegangenen Ansprüche, wobei der Antikörper ein intakter Antikörper mit zwei Paaren von Schwer- und Leichtketten ist.

4. Antikörper zur Verwendung nach einem der vorangegangenen Ansprüche, wobei der Antikörper ein menschlicher κ-IgG1-Antikörper ist.

5. Antikörper zur Verwendung nach einem der vorangegangenen Ansprüche, wobei die Verwendung die Verabreichung des Antikörpers in Kombination mit einem Antikörper gegen BMP9 oder BMP10 umfasst.

6. Antikörper zur Verwendung nach einem der Ansprüche 1 bis 4, wobei die Verwendung die Verabreichung des Antikörpers als Kombinationstherapie mit einem extrazelluläre-Domäne-ACVR1-, -ACVR2A- oder -ACVR2B-Fc-Fusionsprotein oder einem Antikörper gegen Activin A umfasst.

## Revendications

1. Anticorps contre l'Activine B destiné à être utilisé dans une méthode de traitement de la fibrodysplasie ossifiante progressive (FOP), dans lequel l'anticorps contre l'activine B est un anticorps antagoniste.

2. Anticorps à utiliser selon la revendication 1, dans lequel l'anticorps est un anticorps chimérique, plaqué, humanisé ou humain.

3. Anticorps à utiliser selon l'une quelconque des revendications précédentes, dans lequel l'anticorps est un anticorps intact avec deux paires de chaînes lourde et légère.

4. Anticorps à utiliser selon l'une quelconque des revendications précédentes, dans lequel l'anticorps est un anticorps IgG1 kappa humain.

5. Anticorps à utiliser selon l'une quelconque des revendications précédentes, dans lequel l'utilisation comprend l'administration de l'anticorps en combinaison avec un anticorps contre BMP9 ou BMP10.

6. Anticorps à utiliser selon l'une quelconque des revendications 1 à 4, dans lequel l'utilisation comprend l'administration de l'anticorps en thérapie combinée avec une protéine de fusion de domaine Fc extracellulaire ACVR1, ACVR2A ou ACVR2B ou un anticorps contre l'Activine A.
